(19) Europäisches Patentamt
European Patent Office
Office européen des brevets

(11) Veröffentlichungsnummer: **0 197 478**
**B1**

(12) # EUROPÄISCHE PATENTSCHRIFT

(45) Veröffentlichungstag der Patentschrift: **19.09.90**

(21) Anmeldenummer: **86104360.2**

(22) Anmeldetag: **29.03.86**

(51) Int. Cl.[5]: **C 07 F 9/38**, C 07 F 9/40, C 07 K 5/00, A 61 K 31/66, A 61 K 37/02

(54) **Neue Diphosphonsäurederivate, Verfahren zu deren Herstellung und diese Verbindungen enthaltende Arzneimittel.**

(30) Priorität: **06.04.85 DE 3512536**

(43) Veröffentlichungstag der Anmeldung:
**15.10.86 Patentblatt 86/42**

(45) Bekanntmachung des Hinweises auf die Patenterteilung:
**19.09.90 Patentblatt 90/38**

(84) Benannte Vertragsstaaten:
**AT BE CH DE FR GB IT LI LU NL SE**

(56) Entgegenhaltungen:
**EP-A-0 098 567**
**GB-A-2 118 042**

**CHEMICAL ABSTRACTS, Band 90, Nr. 15, 9. April 1979, Seite 47, Zusammenfassung Nr. 115081c, Columbus, Ohio, US; T.A. BANDURINA et al.: "Synthesis of some aminophosphonic acids and their antineoplastic activity", & KHIM.-FARM. ZH. 1978, 12(11), 35-7**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 116**
**D-6800 Mannheim 31 (DE)**

(72) Erfinder: **Bosies, Elmar, Dr. rer. nat.**
**Delpstrasse 11**
**D-6940 Weinheim (DE)**
Erfinder: **Gall, Rudi, Dr. phil.**
**Ulmenstrasse 1**
**D-6945 Hirschberg 1 (DE)**

**EP 0 197 478 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Diphosphonsäurederivate, Verfahren zu deren Herstellung sowie Arzneimittel, die diese Substanzen enthalten.

In der DE—A—18 13 659 sind Diphosphonsäurederivate beschrieben, von denen die 1-Hydroxy-ethan-1.1-diphosphonsäure als Mittel zur Behandlung von Morbus Paget Bedeutung erlangt hat. In der BE—A—896453 sowie der EP—A—96931 sind Aminoalkan-1.1-diphosphonsäuren als gute Calciumkomplexbildner beschrieben, die sich auch zur Behandlung der erhöhten Knockenresorption einsetzen lassen. Solche Substanzen werden jedoch ausgesprochen schlecht resorbiert. Es stellte sich daher die Aufgabe, Aminoalkandiphosphonate mit einer verbesserten Resorption zu finden. Es wurde nun gefunden, daß analogue Derivate dieser Verbindungen, in denen das Stickstoffatom durch Aminosäuren, Di- oder Tripeptide acyliert ist, wesentlich besser resorbiert werden und als ebenso gute Calciumkomplexbildner zur breiteren Behandlung von Calciumstoffwechselstörungen geeignet sind. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a. Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

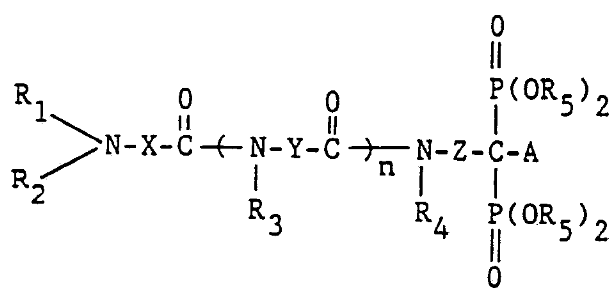

(I)

in der

$R_1$, $R_2$, $R_3$, $R_4$ und $R_5$, jeweils unabhaengig voneinander oder auch gemeinsam Wasserstoff oder $C_1$—$C_4$ Alkyl bedeuten, wobei $R_1$ und X bzw. $R_3$ und Y bzw. $R_4$ und Z zusammen mit dem Stickstoffatom, an das sie gebunden sind, einen Fuenf- oder Sechsring bilden koennen,

X und Y jeweils unabhaengig voneinander eine geradkettige oder verzweigte Alkylenkette mit 1—6 Kohlenstoffatomen, die gegebenenfalls durch Aromaten oder Heteroaromaten substituiert sein kann,

Z eine geradkettige oder verzweigte Alkylenkette mit 1—6 Kohlenstoffatomen, die durch Heteroatome unterbrochen und gegebenenfalls auch durch Aromaten oder Heteroaromaten substituiert sein kann,

n = 0—2 und

A = Wasserstoff oder Hydroxy bedeuten,

sowie deren pharmakologisch unbedenkliche Salze.

Niederes Alkyl bei den Resten $R_1$—$R_5$ bzw. $R_6$—$R_{11}$ bei den Verfahrensvarianten bedeutet Alkylreste mit 1—4 Kohlenstoffatomen, vorzugsweise den Methyl-, Ethyl- und Isopropylrest.

$R_3$ bedeutet vorzugsweise Wasserstoff.

Unter den als Substituenten bei den Resten X, Y und Z angegebenen Aromaten bzw. Heteroaromaten versteht man vorzugsweise den Phenyl- bzw. den Imidazolyl- und Indolylrest.

Als Heteroatome im Rest Z sind vor allem Sauerstoff und Schwefel anzusehen, n bedeutet vorzugsweise 0 und 1.

Die Reste $(R_1R_2)N$—X—CO— bzw. $R_3N$—Y—CO— stellen in der Regel gängige Aminosäurereste dar, wie z.B. Glycin, Alanin, β-Alanin, Valin, Leucin und Isoleucin, bevorzugt sind hierbei vor allem Glycin, Alanin und Leucin, wobei diese Reste gegebenenfalls durch $R_1$, $R_2$ und $R_3$ substituiert sein können.

Falls $(R_1R_2)N$—X—CO— und/oder $R_3N$—Y—CO— einen Ring bilden, stellen diese Reste bevorzugt den Prolinrest dar. Für den Fall, daß $R_4$ und Z zusammen mit dem Stickstoffatom, an das sie gebunden sind, einen Fünfring bzw. Sechsring bilden, stellen diese Reste den 2-Pyrrolidinyl- und 3-Pyrrolidinylrest bzw. einen Piperidinyl-, vorzugsweise den 4-Piperidinylrest dar.

$(R_1R_2)N$—X—CO$(-NR_3$—Y—CO$)_n$ soll für den Fall, daß n = 1 oder 2 bedeutet, vorzugsweise die folgenden Reste darstellen: Gly-Gly, Ala-Ala, Gly-Ala, Ala-Gly oder Gly-Gly-Gly und Ala-Ala-Ala.

In X, Y oder Z vorkommende asymmetrische Kohlenstoffatome können die R-, S- oder R,S-Konfiguration besitzen.

Verbindungen der allgemeinen Formel I werden nach an sich bekannten Verfahren hergestellt, vorzugsweise, indem man

a) ein Diphosphonat der allgemeinen Formel II

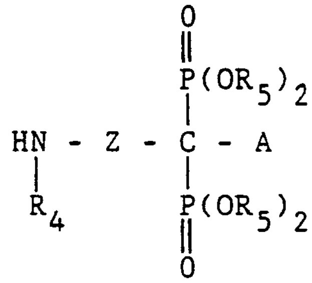

(II)

in der $R_4$, $R_5$, Z und A die oben angegebenen Bedeutungen haben und evtl. im Rest Z vorkommende Stickstoffatome geschuetzt sind,

mit einer aktivierten Carbonsaeure der allgemeinen Formel III

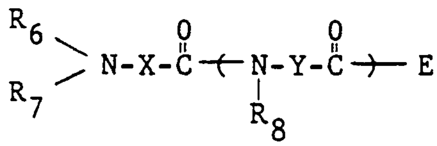

(III)

in der X, Y und n die oben angegebenen Bedeutungen haben, und evtl. in diesen Resten vorkommende Stickstoffatome geschuetzt sind, $R_6$, $R_7$ und $R_8$ Wasserstoff, niederes Alkyl oder eine in der Peptidchemie gaengige Schutzgruppe darstellen, wobei $R_6$ und $R_7$ zusammen auch den Phthaloylrest und $R_6$ und X sowie $R_8$ und Y zusammen mit dem Stickstoffatom, an das sie gebunden sind, einen Fuenf- oder Sechsring bilden koennen, und E die Hydroxy- oder eine in der Peptidchemie gaengige Aktivierungsgruppe darstellt, umsetzt und anschließend die vorhandenen Schutzgruppen abspaltet, oder

b) eine Verbindung der allgemeinen Formel IV

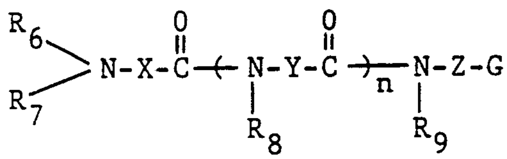

(IV)

in der X, Y, Z und n die oben angegebenen Bedeutungen haben und evtl. in diesen Resten vorkommende Stickstoffatome geschuetzt sind, $R_6$, $R_7$, $R_8$ und $R_9$ Wasserstoff, niederes Alkyl oder eine in der Peptidchemie gaengige Schutzgruppe darstellen, wobei $R_6$ und $R_7$ zusammen auch den Phthaloylrest und $R_6$ und X, $R_8$ und Y sowie $R_9$ und Z zusammen mit dem Stickstoffatom, an das sie gebunden sind, einen Fuenf- oder Sechsring bilden koennen, und G die Gruppe

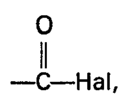

wobei Hal, Brom oder Chlor sein soll oder einen reaktiven Rest, wie z.B. Halogen oder Sulfonat bedeutet, im Falle, daß die Gruppe

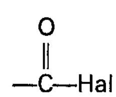

darstellt, mit einem Trialkylphosphit der allgemeinen Formel V

$P(OR_{10})_3$ (V),

in der $R_{10}$ niederes Alkyl bedeutet, zu einem Acrylphosphonat der allgemeinen Formel VI

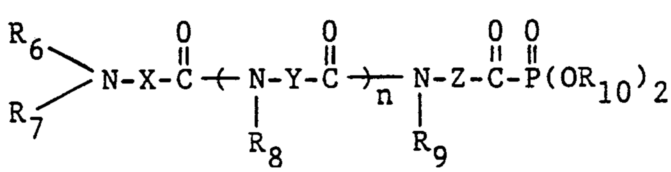

(VI),

in der X, Y, Z, n, $R_6$, $R_7$, $R_8$, $R_9$ und $R_{10}$ die oben angegebenen Bedeutungen haben, umsetzt, anschließend mit einem Dialkylphosphit der allgemeinen Formel VII

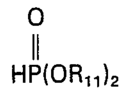

(VII),

in der $R_{11}$ niederes Alkyl bedeutet, zu einem Diphosphonat der allgemeinen Formel VIII

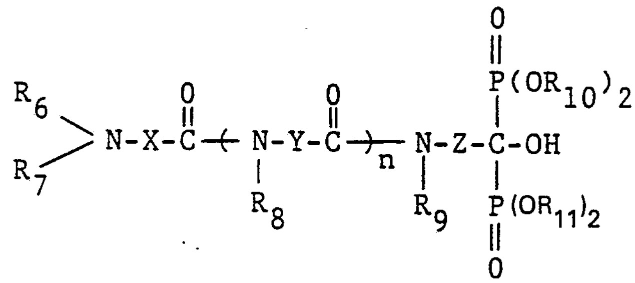

(VIII),

in der X, Y, Z, n, $R_6$, $R_7$, $R_8$, $R_9$, $R_{10}$ und $R_{11}$ die oben angegebenen Bedeutungen haben, reagieren läßt, bzw im Falle, daß G die oben genannte reaktive Gruppe darstellt, mit einer Verbindung der allgemeinen Formel IX

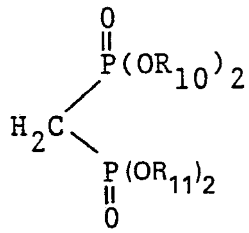

(IX)

in der $R_{10}$ und $R_{11}$ die oben angegebenen Bedeutungen haben, zu einem Diphosphonat der allgemeinen Formel X

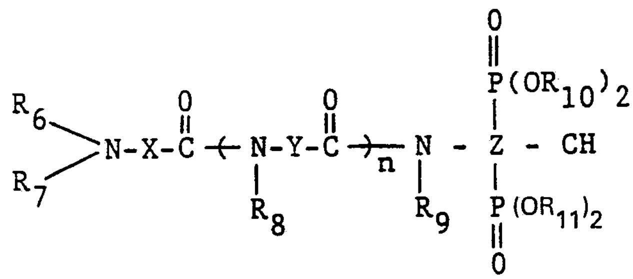

(X),

in der X, Y, Z, n, $R_6$, $R_7$, $R_8$, $R_9$, $R_{10}$ und $R_{11}$ die oben angegebenen Bedeutungen haben, umsetzt, die gegebenenfalls vorhandenen Schutzgruppen abspaltet und gewuenschtenfalls die entstandenen Tetraester zu Diestern oder Saeuren der allgemeinen Formel I verseift.

Die in den oben erlaeuterten Verfahren verwendeten Schutzgruppen sind aus der Peptidchemie bekannt und z.B. in Houben/Weyl Band 15/1 ausfuerhlich beschrieben. Bevorzugt werden als Schutzgruppen Aralkyloxycarbonylgruppen, insbesondere Benzyloxycarbonyl, oder Alkoxycarbonylgruppen, bevorzugt tert.-Butyloxycarbonyl. Es kann jedoch auch eine Formyl-, Trityl-, Trifluoracetyl- oder die Trichlorethoxycarbonylgruppe verwendet werden.

Die Abspaltung der Schutzgruppen nach erfolgter Umsetzung kann in ueblicher Weise durchgefuehrt werden. Benzyloxycarbonyl- und Tritylgruppen lassen sich durch katalytische Hydrierung in Gegenwart von Edelmetallkatalysatoren wie z.B. Palladium auf Kohle, tert.-Butoxycarbonylgruppen durch Einwirkung von starken Saeuren wie z.B. Salzsaeure oder Trifluoressigsaeure abspalten. Trichlorethoxycarbonylgruppen sind reduktive, z.B. durch Einwirkung von Zink in Eisessig spaltbar. Die Phthaloylgruppe laeßt sich in saurem Medium oder auch durch Hydrazinolyse entfernen.

Die in Verfahren a) angegebene Gruppierung

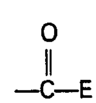

kann ein reaktives Derivat einer Carbonsaeure darstellen. Hierfuer kommen gemischte Anhydride,

insbesondere mit Kohlensaeuremononiederalkylestern wie Ethyl- oder Isobutylestern, oder aktive Ester, insbesondere p-Nitrophenyl-, 2,4,5-Trichlorphenyl-, N-Hydroxysuccinimid- oder 1-N-Hydroxybenzotriazolester in Betracht. Stellt E die Hydroxygruppe dar, kann die Aktivierung der Carboxylgruppe nach dem Carbodiimidverfahren durchgefuehrt werden.

Als reaktiven Rest G im Verfahren b) versteht man Halogen, vorzugsweise das entsprechende Jodid bzw. Bromid oder ein Sulfonat wie z.B. das Mesylat, Benzolsulfonat oder p-Tolyolsulfonat.

Bei Verfahren a) werden vorzugsweise inerte organische Loesungsmittel wie z.B. Methylenchlorid, Aceton, Acetonitril, Dimethylformamid, Tetrahydrofuran oder Dioxan oder Mischungen dieser Loesungsmittel bei Temperaturen von −70 bis +100°C, bevorzugt zwischen −30 und +20°C verwendet. Werden bei der Umsetzung die Phosphonsaeuregruppen durch Salzbildung geschuetzt, so werden die Reaktionen vorzugsweise in waessrigem Medium, evtl. unter Zusatz von Alkoholen oder veretherten Diolen wie z.B. Dimethoxyethan oder in einem Zweiphasensystem wie z.B. Wasser/Methylenchlorid durchgefuehrt, wenn als reaktives Derivat der Verbindung der allgemeinen Formel III ein Carbonsaeurehalogenid, ein gemischtes Anhydrid oder ein Aktivester, vorzugsweise der N-Hydroxysuccinimidester verwendet wird.

Bei Verfahren b) laeßt man fuer den Fall, daß G die Gruppe

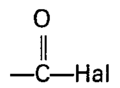

bedeutet, das Saeurechlorid der allgemeinen Formel IV mit dem Trialkylphosphit der allgemeinen Formel V bei Temperaturen zwischen 0 und +80°C, vorzugsweise bei 20—40°C zur Reaktion kommen. Man kann ohne Loesungsmittel oder auch in Gegenwart von inerten Loesungsmittel oder auch in Gegenwart von inerten Loesungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel VI kann isoliert oder direkt weiter umgesetzt werden. Die anschließende Reaktion fuehrt man in Gegenwart einer schwachen Base, vorzugsweise einem sec. Amin wie z.B. Dibutylamin bei einer Temperatur von 0 bis +60°C, vorzugsweise bei 10—30°C durch. Fuer den Fall, daß G in der Verbindung der allgemeinen Formel IV einen reaktiven Rest wie z.B. Halogen oder Sulfonat darstellt, setzt man den Methylendiphosphonsaeureester der allgemeinen Formel IX in Form seines Natrium- oder Kaliumsalzes ein. Hierzu wird er mit Natrium bzw. Kalium oder dem entsprechenden Hybrid in einem inerten Loesungsmittel wie z.B. Benzol, Toluol oder Dimethylformamid umgesetzt. Man kann das Alkalisalz jedoch auch durch Einwirkung eines Alkalialkoholats in dem entsprechenden Alkohol erzeugen und fuehrt die weitere Reaktion mit diesem Alkohol als Loesungsmittel durch. Die Temperaturen liegen hierbei bei 0 bis +40°C, vorzugsweise bei 25°C. Das Alkalisalz wird ohne Isolierung bei Temperaturen zwischen 20 und 110°C mit dem entsprechenden Halogenid bzw. Sulfonat zur Reaktion gebracht.

Die bei den Verfahren a) und b) gegebenenfalls anfallenden Tetraalkylester koennen zu Diestern oder den freien Tetrasaeuren verseift werden. Die Verseifung zu Diestern geschieht in der Regel dadurch, daß man den Tetraalkylester mit einem Alkalihalogenid, vorzugsweise Natriumjodid in einem geeigneten Loesungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disaeure ungewandelt werden kann. Die Verseifung zu freien Diphosphonsaeuren geschieht in der Regel durch Kochen mit Salz- oder Bromwasserstoffsaeure. Man kann jedoch auch eine Spaltung mit Trimethylsilylhalogenid, vorzugsweise dem Bromid oder Jodid vornehmen. Die freien Diphosphonsaeuren koennen umgekehrt durch Kochen mit Orthoameisensaeurealkylestern wieder in die Tetraalkylester ueberfuehrt werden. Die freien Diphosphonsaeuren der allgemeinen Formel I koennen als freie Saeuren oder in Form ihrer Mono- oder Dialkalisalze isoliert werden. Die Alkalisalze lassen sich in der Regent durch Umfaellen aus Wasser/Methanol oder Wasser/Aceton gut reinigen.

Als pharmakologisch vertraegliche Salze werden vor allem Alkali- oder Ammoniumsalze verwendet, die man in ueblicher Weise z.B. durch Neutralisation der Verbindungen mit anorganischen oder organischen Basen wie z.B. Natrium- oder Kaliumhydrogencarbonat, Natronlauge, Kalilauge, waessrigem Ammoniak oder Aminen wie z.B. Trimethyl- oder Triethylamin herstellt.

Die erfindungsgemaeßen neuen Substanzen der Formel I und ihre Salze koennen in fluessiger oder fester Form enteral und parenteral appliziert werden. Hierbei kommen alle ueblichen Applikationsformen in Frage, beispielsweise Tabletten, Kapseln, Dragees, Sirupe, Loesungen, Suspensionen etc. Als Injektionsmedium kommt vorzugsweise Wasser zur Anwendung, welches die bei Injektionsloesungen ueblichen Zusaetze wie Stabilisierungsmittel, Loesungsvermittler und Puffer enthaelt. Derartige Zusaetze sind z.B. Tartrat- und Citrat-Puffer, Ethanol, Komplexbildner (wie Ethylendiamintetraessigsaeure und deren nichttoxische Salze), hochmolekulare Polymere (wie fluessiges Polyethyenoxid) zur Viskositaetsregelung.

Fluessige Traegerstoffe fuer Injektionsloesungen muessen steril sein und werden vorzugsweise in Ampullen abgefuellt. Feste Traegerstoffe sind z.B. Staerke, Lactose, Mannit, Methylcellulose, Talkum, hochdisperse Kieselsaeuren, hoehermolekulare Fettsaeuren (wie Stearinsaeure), Gelatine, Agar-Agar, Calciumphosphat, Magnesiumstearat, tierische und pflanzliche Fette, feste hochmolekulare Polymere (wie Polyethylenglykole); fuer orale Applikation geeignete Zubereitungen koennen gewuenschtenfalls Geschmacks- und Sueßstoffe enthalten.

Die Dosierung kann von verschiedenen Faktoren, wie Applikationsweise, Spezies, Alter und/oder individuellem Zustand abhaengen. Die taeglich zu verabreichenden Dosen liegen bei etwa 1—1000 mg/Mensch, vorzugsweise bei 10—200 mg/Menschund koennen auf einmal oder mehrere Male verteilt eingenommen werden.

Bevorzugt im Sinne der vorliegenden Erfindung sind außer den in den Beispielen genannten Verbindungen und den durch Kombination aller in den Anspruechen genannten Bedeutungen ableitbaren Verbindungen die folgenden Diphosphonate:

2-(N-Glycyl)aminoethan-1-hydroxy-1.1-diphosphonsaeure
2(N-L-Alanyl)aminoethan-1-hydroxy-1.1-diphosphonsaeure
2-(N-L-Prolyl)aminoethan-1-hydroxy-1.1-diphosphonsaeure
2-(N-Sarkosyl)aminoethan-1-hydroxy-1.1-diphosphonsaeure
2-(N-L-Valyl)aminoethan-1-hydroxy-1.1-diphosphonsaeure
2-(N-Glycylglycyl)aminoethan-1-hydroxy-1.1-diphosphonsaeure
1-Hydroxy-1-[(N-Glycyl)piperidin-4-yl]methan-1.1-diphosphonsäure
3-(N-L-Leucyl)aminopropan-1-hydroxy-1.1-diphosphonsaeure
3-(N-Glycylglycyl)aminopropan-1-hydroxy-1.1-diphosphonsaeure
3-(N-Alanylglycyl)aminopropan-1-hydroxy-1.1-diphosphonsaeure
3-(N-Glycylglycylglycyl)aminopropan-1-hydroxy-1.1-diphosphonsaeure
4-(N-L-Prolyl)aminobutan-1-hydroxy-1.1-diphosphonsaeure
4-(N-L-Alanylglycyl)aminobutan-1-hydroxy-1.1-diphosphonsaeure
4-(N-L-Alanyl-L-alanyl)aminobutan-1-hydroxy-1.1-diphosphonsaeure
5-(N-L-Valyl)aminopentan-1-hydroxy-1.1-diphosphonsaeure
5-(N-Glycyl-L-Alanyl)aminopentan-1-hydroxy-1.1-diphosphonsaeure
5-(N-L-Alanyl-L-alanyl)aminopentan-1-hydroxy-1.1-diphosphonsaeure
5-(N-L-Alanyl-L-alanyl-L-alanyl)aminopentan-1-hydroxy-1.1-diphosphonsaeure
6-(N-Glycylglycyl)aminohexan-1-hydroxy-1.1-diphosphonsaeure
7-(N-L-Alanyl)aminoheptan-1-hydroxy-1.1-diphosphonsaeure
7-(N-L-Alanyl-L-alanyl)aminoheptan-1-hydroxy-1.1-diphosphonsaeure
R-2-(N-Glycyl)aminopropan-1-hydroxy-1.1-diphosphonsaeure
R-2-(N-L-Alanyl)aminopropan-1-hydroxy-1.1-diphosphonsaeure
R,S-2-(N-Glycylglycyl)aminopropan-1-hydroxy-1.1-diphosphonsaeure
R,S-2-(N-L-Alanylglycyl)aminopropan-1-hydroxy-1.1-diphosphonsaeure
S-2-(N-Glycyl)aminopropan-1-hydroxy-1.1-diphosphonsaeure
S-2-(N-L-Alanyl)aminopropan-1-hydroxy-1.1-diphosphonsaeure
R-4-(N-Glycyl-aminopentan-1-hydroxy-1.1-diphosphonsaeure
S-4-(N-Glycyl]aminopentan-1-hydroxy-1.1-diphosphonsaeure
R,S-4-(N-L-Alanyl)aminopentan-1-hydroxy-1.1-diphosphonsaeure
R,S-4-(N-D-Alanyl)aminopentan-1-hydroxy-1.1-diphosphonsaeure
R,S-4-(N-D,L-Alanyl)aminopentan-1-1-hydroxy-1.1-diphosphonsaeure
R,S-4-(N-L-Prolyl)aminopentan-1-hydroxy-1.1-diphosphonsaeure
R,S-4-(N-L-Leucyl)aminopentan-1-hydroxy-1.1-diphosphonsaeure
R,S-4-(N-Glycylglycyl)aminopentan-1-hydroxy-1.1-diphosphonsaeure
R,S-4-(N-L-Alanyl-L-alanyl)aminopentan-1-hydroxy-1.1-diphosphonsaeure
R,S-4-(N-Glycyl-L-alanyl)aminopentan-1-hydroxy-1.1-diphosphonsaeure
R,S-4-(N-L-Alanylglycyl)aminopentan-1-hydroxy-1.1-diphosphonsaeure
R,S-4-(N-Glycylglycylglycyl)aminopentan-1-hydroxy-1.1-diphosphonsaeure
R-5-(N-Glycyl)aminohexan-1-hydroxy-1.1-diphosphonsaeure
S-5-(N-Glycyl)aminohexan-1-hydroxy-1.1-diphosphonsaeure
R,S-5-(N-Glycyl)aminohexan-1-hydroxy-1.1-diphosphonsaeure
R,S-5-(N-L-Alanyl)aminohexan-1-hydroxy-1.1-diphosphonsaeure
R,S-5-(N-L-Alanylglycyl)aminohexan-1-hydroxy-1.1-diphosphonsaeure
5-(N-Glycyl)amino-4-oxa-pentan-1-hydroxy-1.1-diphosphonsaeure
6-(N-L-Alanyl)amino-4-oxa-hexan-1-hydroxy-1.1-diphosphonsaeure
1-Hydroxy-1-[(N-Glycyl)pyrrolidin-2-yl]methan-1.1-diphosphonsaeure
6-(N-Glycyl)amino-4-thia-hexan-1-hydroxy-1.1-diphosphonsaeure
2-Benzyl-2-(N-glycyl)aminoethan-1-hydroxy-1.1-diphosphonsaeure
2-(N-Glycyl)amino-3-(4-imidazolyl)propan-1-hydroxy-1.1-diphosphonsaeure
2-(N-Glycylglycyl)amino-3-(4-imidazolyl)propan-1-hydroxy-1.1-diphosphonsaeure
2-(N-L-Alanyl)amino-3-(3-indolyl)propan-1-hydroxy-1.1-diphosphonsaeure
2-(N-L-Alanyl-L-alanyl)amino-3-(3-indolyl)propan-1-hydroxy-1.1-diphosphonsaeure
3-(N-Glycyl)aminopropan-1.1-diphosphonsaeure
3-(N-Glycylglycyl)aminopropan-1.1-diphosphonsaeure
3-(N-L-Alanyl)aminopropan-1.1-diphosphonsaeure
4-(N-Glycyl)aminobutan-1.1-diphosphonsaeure
4-(N-L-Alanyl)aminobutan-1.1-diphosphonsaeure

4-(N-L-Prolyl)aminobutan-1.1-diphosphonsaeure
4-(N-Glycyl-L-alanyl)aminobutan-1.1-diphosphonsaeure
5-(N-Glycyl)aminopentan-1.1-diphosphonsaeure
5-(N-L-Alanylglycyl)aminopentan-1.1-diphosphonsaeure
6-(N-Glycyl)aminohexan-1.1-diphosphonsaeure
6-(N-L-Alanyl)aminohexan-1.1-diphosphonsaeure
6-(N-Glycyl)amino-4-oxa-hexan-1.1-diphosphonsaeure
6-(N-L-Prolyl)amino-4-thia-hexan-1.1-diphosphonsaeure
3-(N-Glycyl-N-methyl)aminopropan-1-hydroxy-1.1-diphosphonsaeure
3-(N-L-Alanyl-N-methyl)aminopropan-1-hydroxy-1.1-diphosphonsaeure
3-(N-D-Alanyl-N-methyl)aminopropan-1-hydroxy-1.1-diphosphonsaeure
4-(N-L-Alanyl-N-methyl)aminobutan-1-hydroxy-1.1-diphosphonsaeure
4-(N-D-Alanyl-N-methyl)aminobutan-1-hydroxy-1.1-diphosphonsaeure
1-[(N-L-Alanyl)pyrrolidin-3-yl]methan-1-hydroxy-1.1-diphosphonsaeure

Die nachfolgenden Beispiele zeigen einige der Verfahrensvarianten, die zur Synthese der erfindungsgemaeßen Verbindungen verwendet werden koennen. Die Struktur dieser Verbindungen ist durch H- und P-NMR-Spektroskopie gesichert, die Reinheit wurde mittels P-NMR-Spektroskopie, Duennschichtelektrophorese (Cellulose, Oxalat-Puffer von pH = 4.0) und mittels, C,H,N,P,Na-Analyse bestimmt. Zur Charakterisierung der einzelnen Substanzen werden die $M_{rel}$-Werte (= relative Mobilitaet) bezogen auf Pyrophosphat ($M_{rel}$ = 1.0) angegeben.

## Beispiel 1

4-N-(Glycyl)aminobutan-1-hydroxy-1.1-diphosphonsaeure

4 g Tetranatriumsalz der 4-Aminobutan-1-hydroxy-1.1-disphosphonsaeure werden in 40 ml Wasser geloest und unter Ruehren bei Raumtemperatur portionsweise mit 4 g Phthaloylglycylchlorid versetzt. Durch gleiczeitiges Zutropfen von 1 N Natronlauge haelt man den pH-Wert der Loesung bei 11—12. Nach einstuendigem Nachruehren gibt man soviel Amberlite® IR 120 $H^+$-Form zu, daß der pH-Wert auf 1.4 faellt. Nach Abtrennung des Ionenaustauschers gießt man in 1.6 l Aceton, saugt die ausgefallenen Kristalle ab, waescht mit Aceton und trocknet. Man erhaelt so 4.7 g = 89%, (Fp. 103°C Z., $M_{rel}$: 0.6).

2 g Dinatriumsalz der 1-Hydroxy-4-(N-phthaloyl-glycyl)aminobutan-1.1-disphosphonsaeure werden in 20 ml Wasser geloest, mit 0.5 ml Eisessig und 0.9 ml Phenylhydrazin versetzt und 5 h bei 50°C geruehrt. Nach Abkuehlen und Abtrennen des ausgefallenen Phthaloyl-phenylhydrazids ruehrt man in Methanol ein und erhaelt 1.1 g = 68% der gewuenschten Verbindung in Form des Dinatriumsalzes (Fp. >300°C, $M_{rel}$: 0.36).

## Beispiel 2

Die Abspaltung der Phthalsaeure kann auch durch einstuendiges Kochen von 19 ml einer waessrigen Loesung von 1.9 g 1-Hydroxy-4-(N-phthaloyl-glycyl)aminobutan-1.1-diphosphonsaeure bewirkt werden. Nach Abtrennen der bei Abkuehlen ausfallenden Phthalsaeure engt man das Filtrat ein und ruehrt den Rueckstand mit Methanol aus. Man erhaelt so 1.0 g = 74% *4-(N-Glycyl)aminobutan-1-hydroxy-1.1-diphosphonsaeure* (Fp. 200°C sintern/202—204°C Z., aus Methanol/Wasser; $M_{rel}$: 0.36).

## Beispiel 3

4-(N-Glycylglycyl)aminobutan-1-hydroxy-1.1-disphosphonsaeure

Aus der in Beispiel 1 beschriebenen Verbindung 4-(N-Glycyl)-aminobutan-1-hydroxy-1.1-diphosphonsaeure stellt man in analoger Weise die N-Phthaloylglycylglycylverbindung her (Ausbeute: 65%, Fp. ab 96°C Sintern und langsames Aufschaeumen, $M_{rel}$: 0.55).

Die Abspaltung des Phthalylrestes erfolgt wie in Beispiel 1 beschrieben mit Phenylhydrazin. Nach Abtrennung des ausgefallenen Phthaloylphenylhydrazids ruerht man das Filtrat in der 10fachen Menge Methanol ein, isoliert die ausgefallenen Kristalle, loest sie in Wasser und saeuert mit Ionenaustauscher an. Das Filtrat engt man ein und verruehrt den Rueckstand mit Methanol. Man erhaelt so 27% mit einem Fp. 122°C Sintern/170—172°C Zers.; $M_{rel}$: 0.34.

In analoger Weise erhaelt man durch Umsetzung des Tetranatrium salzes von

a) 4-Aminobutan-1-hydroxy-1.1-diphosphonsaeure mit Phthaloyl-D-alanylchlorid nach Ansaeuern die 1-Hydroxy-4-(N-phthaloyl-D-alanyl)aminobutan-1.1-diphosphonsaeure (Fp. 113°C Sintern, 133—136°C Z; $M_{rel}$: 0.59) in einer Ausbeute von 99% und daraus durch Abspaltung mit Phenylhydrazin in einer Ausbeute von 46% die *4-(N-D-Alanyl)aminobutan-1-hydroxy-1.1-diphosphonsaeure* (Fp. 117°C., $M_{rel}$: 0.37).

b) 4-Aminobutan-1-hydroxy-1.1-diphosphonsaeure mit Phthaloyl-L-alanylchlorid nach Ansaeuern die 1-Hydroxy-4-(N-phthalopyl-L-alanyl)aminobutan-1.1-disphosphonsaeure (Fp. 92°C Sintern, 137—141°C Z, $M_{rel}$: 0.59) in einer Ausbeute von 97% und daraus durch Abspaltung mit Phenylhydrazin in einer Ausbeute von 53% die *4-(N-L-Alanyl)aminobutan-1-hydroxy-1.1-diphosphonsaeure* (Fp. 102°C Z, $M_{rel}$: 0.37).

## Beispiel 4

5-(N-Glycyl)aminopentan-1-hydroxy-1.1-diphosphonsaeure

6.06 g 5-Aminopentan-1-hydroxy-1.1-diphosphonsaeure werden in 80 ml Wasser vorgelegt und mit 10 N Natronlauge auf einen pH = 11 gestellt. Man tropft unter Ruerhen eine Loesung von 6.8 g Phthaloylglycylchlorid in 80 ml Methylenchlorid zu und haelt den pH-Wert der Loesung durch tropfenweise Zugabe

von 10 N Natronlauge zwischen pH = 10 und 11. Nach 3 Stunden werden die Phasen getrennt, die waessrige zweimal mit Methylenchlorid extrahiert und mit 2 N Salzsaeure auf einen pH = 5 gebracht. Die Loesung wird mit 350 ml Methanol versetzt und mehrere Stunden unter Eiskuehlung geruehrt. Der Niederschlag wird abgesaugt und mit Methanol gewaschen. Man erhaelt 6.7 g = 65% des Dinatriumsalzes der 1-Hydroxy-5-(N-phthaloylglycyl)aminopentan-1.1-diphosphonsaeure, Fp. >300°C, $M_{rel}$: 0.55.

3.07 g dieses Dinatriumsalzes loest man in 30 ml Wasser, versetzt mit 1.27 g Phenylhydrazin und 720 mg Essigsaeure und erhitzt 30 Stunden auf 50°C. Nach dem Abkuehlen wird der Niederschlag abgesaugt, das Filtrat mit Aktivkohle behandelt und langsam mit 90 ml Methanol versetzt. Man ruehrt 1 Stunde unter Eiskuehlung und saugt den Niederschlag ab. Man erhaelt 1.6 g = 70% des Mononatriumsalzes der *4-(N-Glycyl)aminopentan-1-hydroxy-1.1-diphosphonsaeure,* Fp. ≈250°C Z, $M_{rel}$: 0.40.

In analoger Weise erhaelt man durch Umsetzung des

a) 5-Aminopentan-1-hydroxy-1.1-diphosphonats mit Phthaloyl-D,L-alanylchlorid das Dinatriumsalz der 1-Hydroxy-5-(N-phthaloyl-D,L-alanyl)aminopentan-1.1-diphosphonsaeure (Fp. >300°C; $M_{rel}$: 0.60; in einer Ausbeute von 65% und daraus durch Abspaltung mit Phenylhydrazin in einer Ausbeute von 52% das Mononatriumsalz der *5-(N-D,L-Alanyl-aminopentan-1-hydroxy-1.1-diphosphonsaeure* (Fp. ≈270°C Z., $M_{rel}$: 0.38).

b) 5-Amino-1-hydroxy-1.1-diphosphonats mit Phthaloyl-β-alanylchlorid das Dinatriumsalz der 1-Hydroxy-5-(N-phthaloyl-β-alanyl)aminopentan-1.1-diphosphonsaeure (Fp. >300°C, $M_{rel}$: 0.60) in einer Ausbeute von 50% und daraus durch Abspaltung mit Phenylhydrazin in einer Ausbeute von 59% das Mononatriumsalz der *5-(N-β-Alanyl)aminopentan-1-hydroxy-1.1-diphosphonsaeure* (Fp. 260°C Z., $M_{rel}$: 0.35).

Beispiel 5

5-(N-Glycyl)aminopentan-1-hydroxy-1.1-diphosphonsaeure

0.75 g 5-Aminopentan-1-hydroxy-1.1-diphosphonsaeure-dinatriumsalz werden in 50 ml Wasser geloest und mit 0.21 g Natriumhydrogencarbonat versetzt. Anschließend gibt man unter Ruehren 1.53 g N-Benzyloxycarbonyl-glycin-(N-hydroxysuccinimid)ester in 10 ml Dimethoxyethan zu und haelt den pH-Wert der Loesung durch Zugabe von festem Natriumhydrogencarbonat zwischen 7.0 und 7.5. Nach 1 Stunde wird von Ungeloestem abfiltriert, das Filtrat mit 2 N Salzsaeure auf einem pH = 5 gestellt und mit 300 ml Methanol versetzt. Man kuehlt, saugt den Niederschlag ab und erhaelt 0.92 g ≙71% der 5-(N-Benzyloxycarbonylglycyl)aminopentan-1-hydroxy-1.1-diphosphonsaeure als Natriumsalz (Fp. >300°C, $M_{rel}$: 0.58).

Diese 0.92 g loest man in 70 ml Wasser/35 ml Ethanol, gibt 1 g Palladium auf Kohle (10proz.) zu und hydriert unter Normaldruck bei Zimmertemperatur. Nach Beendigung der Wasserstoffaufnahme wird der Katalysator abgesaugt und das Filtrat mit 300 ml Aceton versetzt. Man saugt den Niederschlag ab und erhaelt 0.41 g ≙50% der *5-(N-Glycyl)aminopentan-1-hydroxy-1.1-diphosphonsaeure* als Dinatriumsalz (Fp. 240°C Sintern, 285°C Z.; $M_{rel}$: 0.40).

In analoger Weise erhaelt man durch Umsetzung der

a) 5-Aminopentan-1-hydroxy-1.1-diphosphonsaeure mit dem N-Benzyloxycarbonyl-L-alanin-(N-hydroxysuccinimid)ester und anschließender Hydrierung die *5-(N-L-Alanyl)aminopentan-1-hydroxy-1.1-disphosphonsaeure* als Dinatriumsalz in einer Ausbeute von 73% (Fp. >300°C, $M_{rel}$: 0.38, $[\alpha]_D^{20}$: +16.1°, c = 2.5 in Wasser).

b) 5-Aminopentan-1-hydroxy-1.1-diphosphonsaeure mit dem N-Benzyloxycarbonyl-D-alanin-(N-hydroxysuccinimid)ester und anschließender Hydrierung die *5-(N-D-Alanyl)aminopentan-1-hydroxy-1.1-diphosphonsaeure* als Dinatriumsalz in einer Ausbeute von 68% (Fp. >300°C, $M_{rel}$: 0.38, $[\alpha]_D^{20}$: −15.8°, c = 2.5 in Wasser).

c) 3-Aminopropan-1-hydroxy-1.1-diphosphonsaeure mit dem N-Benzyloxycarbonyl-glycin-(N-hydroxysuccinimid)ester und anschließender Hydrierung die *3-(N-Glycyl-Aminopropan-1-hydroxy-1.1-diphosphonsaeure,* die als freie Base isoliert wurde, in einer Ausbeute von 56% (Fp. 120°C Sintern 155—160°C, $M_{rel}$: 0,42).

d) 3-Aminopropan-1-hydroxy-1.1-diphosphonsaeure mit dem N-Benzyloxycarbonyl-L-alanin-(N-hydroxysuccinimid)ester und anschließender Hydrierung die *3-(N-L-Alanyl)aminopropen-1-hydroxy-1.1-diphosphonsaeure* als Dinatriumsalz in einer Ausbeute von 52% (Fp. >300°C, $M_{rel}$: 0.35, $[\alpha]_D^{20}$: +13.2°, c = 2.5 in Wasser).

e) 3-Aminopropan-1-hydroxy-1.1-diphosphonsaeure mit dem N-Benzyloxycarbonyl-D-alanin-(N-hydroxysuccinimid)ester und anschließender Hydrierung die *3-(N-D-Alanyl)aminopropan-1-hydroxy-1.1-diphosphonsaeure* als Dinatriumsalz in einer Ausbeute von 57% (Fp. >300°C, $M_{rel}$: 0.35, $[\alpha]_D^{20}$: −13.7°, c = 2.5 in Wasser).

Beispiel 6

In analoger Weise zu Beispiel 5 erhaelt man durch Umsetzung der

a) 3-Aminopropan-1-hydroxy-1.1-diphosphonsaeure mit dem N-Benzyloxycarbonyl-L-prolin-(N-hydroxysuccinimid)ester und anschließender Hydrierung die *3-(N-L-Prolyl)aminopropan-1-hydroxy-1.1-diphosphonsaeuer* als Dinatriumsalz in einer Ausbeute von 21% (Fp. >300°C; $M_{rel}$: 0.20).

b) 5-Aminopentan-1-hydroxy-1.1-diphosphonsaeure mit dem N-Benzyloxycarbonyl-L-prolin-(N-hydroxysuccinimid) ester und anschließender Hydrierung die *5-(N-L-Prolyl)aminopentan-1-hydroxy-1.1-diphosphonsaeure* als Dinatriumsalz in einer Ausbeute von 52% (Fp. >300°C, $M_{rel}$: 0.38).

c) 5-Aminopentan-1-hydroxy-1.1-diphosphonsaeure mit dem N-Benzyloxycarbonyl-glycylglycin-(N-hydroxysuccinimid)ester (Fp. 155—158°C) und anschließender Hydrierung die *5-(N-Glycylglycyl)aminopentan-1-hydroxy-1.1-diphosphonsaeure* als Dinatriumsalz in einer Ausbeute von 54% (Fp. >300°C; $M_{rel}$: 0.32).

d) 3-Aminopropan-1-hydroxy-1.1-diphosphonsaeure mit dem N-Benzyloxycarbonyl-L-alanyl-L-alanin(N-hydroxysuccinimid)ester (Fp. 150—153°C) und anschließender Hydrierung die *3-(N-L-Alanyl-L-Alanyl)aminopropan-1-hydroxy-1.1-disphosphonsaeure* als Dinatriumsalz in einer Ausbeute von 14% (Fp. >300°C; $M_{rel}$: 0.25).

e) 4-Aminobutan-1-hydroxy-1.1-diphosphonsaeure mit dem N-Benzyloxycarbonyl-L-prolin)N-hydroxysuccinimid) ester und anschließender Hydrierung die *4-(N-L-Prolyl)aminobutan-1-hydroxy-1.1-diphosphonsaeure* in einer Ausbeute von 45% (Fp. 75°C Z.; $M_{rel}$: 0.20).

f) 4-Aminobutan-1-hydroxy-1.1-diphosphonsaeure mit den N-Benzyloxycarbonyl-sarkosin(N-hydroxysuccinimid)ester und anschließender Hydrierung die *4-(N-Sarkosyl)aminobutan-1-hydroxy-1.1-diphosphonsaeure* in einer Ausbeute von 50% (Fp. 97—101°C Z; $M_{rel}$, 0.26).

Beispiel 7

In analoger Weise zu Beispiel 1 erhaelt man durch Umsetzung der

a) 4-(N-Methyl)aminobutan-1-hydroxy-1.1-diphosphonsaeure mit Phthaloylglycylchlorid und anschließender Abspaltung der Phthaloylgruppe mit Wasser (wie in Beispiel 2 beschrieben) die *4-(N-Glycyl-N-methyl)aminobutan-1-hydroxy-1.1-diphosphonsaeure* in einer Ausbeute von 52% (Fp. 205—201°C Z; $M_{rel}$: 0.34).

b) 4-Aminobutan-1-hydroxy-1.1-diphosphonsaeure mit Phthaloylaminoisobuttersaeurechlorid (Fp. 78—81°C) und anschließender Abspaltung der Phthaloylgruppe die *4-(N-Aminoisobutyryl)aminobutan-1-hydroxy-1.1-diphosphonsaeure* in einer Ausbeute von 12% (Fp. 80°C Z; $M_{rel}$: 0.24).

c) 6-Aminohexan-1-hydroxy-1.1-diphosphonsaeure mit Phthaloylglycylchlorid und anschließender Abspaltung der Phthaloylgruppe die *6-(N-Glycyl)aminohexan-1-hydroxy-1.1-diphosphonsaeure* in einer Ausbeute von 13% (Fp. 165—170°C Z; $M_{rel}$: 0.31.

d) 4-(N-(Glycylglycyl)aminobutan-1-hydroxy-1.1-disphosphonsaeure (siehe Beispiel 3) mit Phthaloylglycylchlorid und anschließender Abspaltung der Phthaloylgruppe die *4-(N-Glycylglycylglycyl)aminobutan-1-hydroxy-1.1-diphosphonsaeure* in einer Ausbeute von 6% (Fp. 100—103°C Z; $M_{rel}$: 0.21).

**Patentansprüche**

1. Diphosphonsaeure-Derivate der allgemeinen Formel I

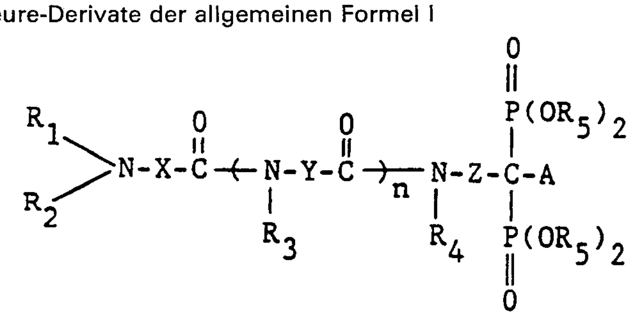

(I)

in der

$R_1$, $R_2$, $R_3$, $R_4$ und $R_5$, jeweils unabhaengig voneinander oder auch gemeinsam Wasserstoff oder $C_1$—$C_4$ Alkyl bedeuten, wobei $R_1$ und X bzw. $R_3$ und Y bzw. $R_4$ und Z zusammen mit dem Stickstoffatom, an das sie gebunden sind, einen Fuenf- oder Sechsring bilden koennen,

X und Y jeweils unabhaengig voneinander eine geradkettige oder verzweigte Alkylenkette mit 1—6 Kohlenstoffatomen, die gegebenenfalls durch Aromaten oder Heteroaromaten substituiert sein kann,

Z eine geradkettige oder verzweigte Alkylenkette mit 1—6 Kohlenstoffatomen, die durch Heteroatome unterbrochen und gegebenenfalls auch durch Aromaten oder Heteroaromaten substituiert sein kann,

n = 0—2 und

A = Wasserstoff oder Hydroxy bedeuten,

sowie deren pharmakologisch unbedenkliche Salze.

2. Verbindungen gemäß Anspruch 1, in denen A Hydroxy, n die Zahl 0 oder 1, $R_4$ und $R_5$ Wasserstoff, Z eine geradkettige Alkylenkette mit 2—5 Kohlenstoffatomen, die Gruppe ($R_1R_2$)—N—X—CO— Glycyl, Alanyl, Prolyl oder Sarkosyl und die Gruppe

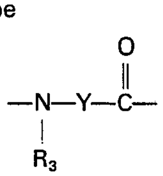

Glycyl oder Analyl bedeuten.

3. Verfahren zur Herstellung von Diphosphonsaeure-Derivaten der allgemeinen Formel I

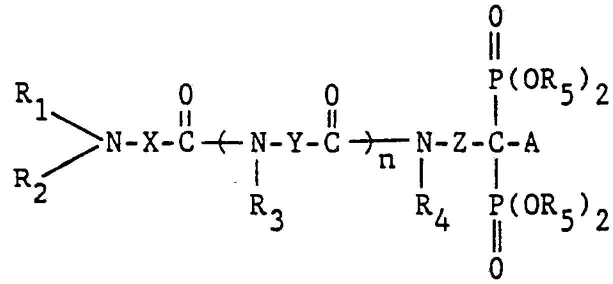

(I)

in der

$R_1$, $R_2$, $R_3$, $R_4$ und $R_5$, jeweils unabhaengig voneinander oder auch gemeinsam Wasserstoff oder $C_1$—$C_4$ Alkyl bedeuten, wobei $R_1$ und X bzw. $R_3$ und Y bzw. $R_4$ und Z zusammen mit dem Stickstoffatom, an das sie gebunden sind, einen Fuenf- oder Sechsring bilden koennen,

X und Y jeweils unabhaengig voneinander eine geradkettige oder verzweigte Alkylenkette mit 1—6 Kohlenstoffatomen, die gegebenenfalls durch Aromaten oder Heteroaromaten substituiert sein kann,

Z eine geradkettige oder verzweigte Alkylenkette mit 1—6 Kohlenstoffatomen, die durch Heteroatome unterbrochen und gegebenenfalls auch durch Aromaten oder Heteroaromaten substituiert sein kann,

n = 0—2 und

A = Wasserstoff oder Hydroxy bedeuten,

sowie deren pharmakologisch unbedenkliche Salze, dadurch gekennzeichnet, daß man

a) ein Diphosphonat der allgemeinen Formel II

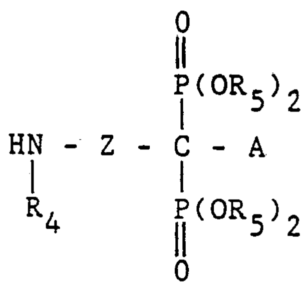

(II)

in der $R_4$, $R_5$, Z und A die oben angegebenen Bedeutungen haben und evtl. im Rest Z vorkommende Stickstoffatome geschuetzt sind, mit einer aktivierten Carbonsaeure der allgemeinen Formel III

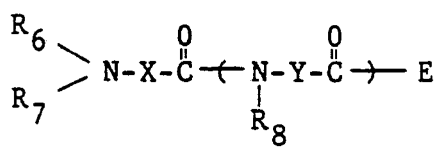

(III)

in der X, Y und n die oben angegebenen Bedeutungen haben, und evtl. in diesen Resten vorkommende Stickstoffatome geschuetzt sind, $R_6$, $R_7$ und $R_8$ Wasserstoff, niederes Alkyl oder eine in der Peptidchemie gaengige Schutzgruppe darstellen, wobei $R_6$ und $R_7$ zusammen auch den Phthaloylrest und $R_6$ und X sowie $R_8$ und Y zusammen mit dem Stickstoffatomen, an das sie gebunden sind, einen Fuenf- oder Sechsring bilden koennen, und E die Hydroxy- oder eine in der Peptidchemie gaengige Aktivierungsgruppe darstellt, umsetzt und anschließend die vorhandenen Schutzgruppen abspaltet, oder

b) eine Verbindung der allgemeinen Formel IV

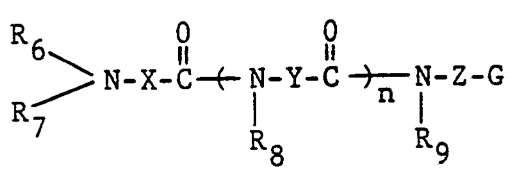

(IV)

in der X, Y, Z und n die oben angegebenen Bedeutungen haben und evtl. in diesen Resten vorkommende Stickstoffatome geschuetzt sind, $R_6$, $R_7$, $R_8$ und $R_9$ Wasserstoff, niederes Alkyl oder eine in der Peptidchemie gaengige Schutzgruppe darstellen, wobei $R_6$ und $R_7$ zusammen auch den Phthaloylrest und $R_6$ und X, $R_8$ und Y sowie $R_9$ und Z zusammen mit dem Stickstoffatom, an das sie gebunden sind, einen Fuenf- oder Sechsring bilden koennen, und G die Gruppe

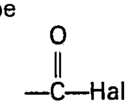

wobei Hal Brom oder Chlor sein soll oder einen reaktiven Rest wie z.B. Halogen oder Sulfonat bedeutet, im Falle, daß G die Gruppe

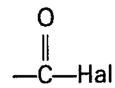

darstellt, mit einem Trialkylphosphit der allgemeinen Formel V

$$P(OR_{10})_3 \qquad (V),$$

in der $R_{10}$ niederes Alkyl bedeutet, zu einem Acylphosphonat der allgemeinen Formel VI

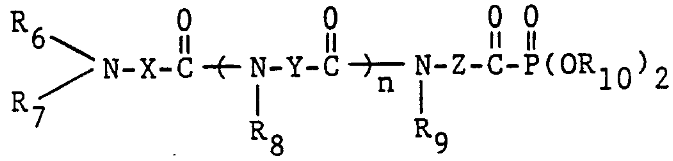

(VI),

in der X, Y, Z, n, $R_6$, $R_7$, $R_8$, $R_9$ und $R_{10}$ die oben angegebenen Bedeutungen haben, umsetzt, anschließend mit einem Dialkylphosphit der allgemeinen Formel VII

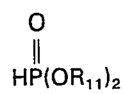

(VII),

in der $R_{11}$ niederes Alkyl bedeutet, zu einem Diphosphonat der allgemeinen Formel VIII

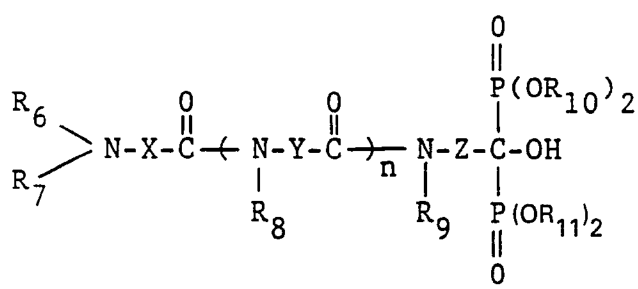

(VIII),

in der X, Y, Z, n, $R_6$, $R_7$, $R_8$, $R_9$, $R_{10}$ und $R_{11}$ die oben angegebenen Bedeutungen haben, reagieren läßt, bzw im Falle, daß G die oben genannte reaktive Gruppe darstellt, mit einer Verbindung der allgemeinen Formel IX

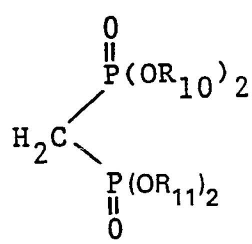

(IX)

in der $R_{10}$ und $R_{11}$ die oben angegebenen Bedeutungen haben, zu einem Diphosphonat der allgemeinen Formel X

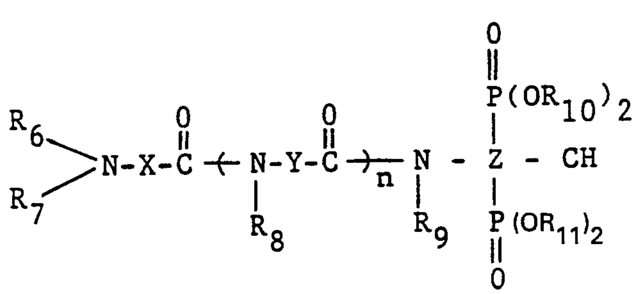

(X),

in der X, Y, Z, n, $R_6$, $R_7$, $R_8$, $R_9$, $R_{10}$ und $R_{11}$ die oben angegebenen Bedeutungen haben, umsetzt, die gegebenenfalls vorhandenen Schutzgruppen abspaltet und gewuenschtenfalls die entstandenen

Tetraester zu Diestern oder Saeuren der allgemeinen Formel I verseift oder in pharmakologisch unbedenkliche Salze ueberfuehrt.

4. Verfahren gemaeß Anspruch 3, dadurch gekennzeichnet, daß Verbindungen der Formel I hergestellt werden, in denen A Hydroxy, n die Zahl 0 oder 1, $R_4$ und $R_5$ Wasserstoff, Z eine geradkettige Alkylenkette mit 2—5 Kohlenstoffatomen, die Gruppe $(R_1R_2)N—X—CO—$ Glycyl, Alanyl, Prolyl oder Sarkosyl und die Gruppe

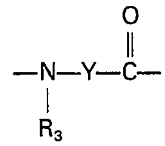

Glycyl oder Alanyl bedeuten.

5. Arzneimittel, enthaltend eine Verbindung gemaeß Anspruch 1 und uebliche Traeger- und Hilfsstoffe.

6. Verwendung von Verbindungen gemaeß Anspruch 1 oder Herstellung von Arzneimitteln zur Behandlung von Calciumstoffwechselstoerungen.

**Revendications**

1. Dérivés d'acides diphosphoniques de formule générale I

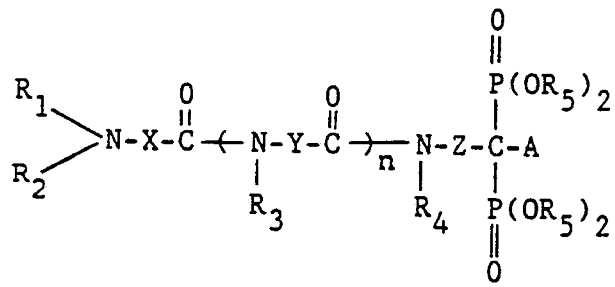

(I)

où

$R_1$, $R_2$, $R_3$, $R_4$ et $R_5$ représentent, indépendamment ou tous ensemble un atome d'hydrogène ou un groupe alkyle en $C_1—C_4$, $R_1$ et X pouvent former, ainsi que $R_3$ et Y ou $R_4$ et Z, conjointement avec l'atome d'azote auquel ils sont liés, un cycle à cinq ou six maillons,

X et Y représentent indépendamment une chaîne alkylène linéaire ou ramifiée, comportant 1 à 6 atomes de carbone, qui peut être éventuellement substituée par des groupes aromatiques ou hétéroaromatiques,

Z représente une chaîne alkylène linéaire ou ramifiée, comportant 1 à 6 atomes de carbone, qui peut être interrompue par des hétéroatoms et éventuellement aussie substituée par des groupes aromatiques ou hétéromatiques,

n = 0—2 et

A = un atome d'hydrogène ou un groupe hydroxy,

ainsi que leurs sels pharmaceutiquement acceptables.

2. Composés selon la revendication 1, dans lesquels A représente un groupe hydroxy, n vaut 0 ou 1, $R_4$ et $R_5$ représentent un atome d'hydrogène, Z représente une chaîne alkylène linéaire comportant 2—5 atomes de carbone, le groupe $(R_1R_2)—N—X—CO—$ représente un groupe glycyle, alanyle, prolyle ou sarcosyle, et le groupe

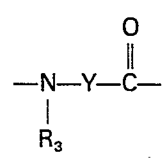

représente un groupe glycyle ou alanyle.

3. Procédé pour la préparation de dérivés d'acides diphosphoniques de formule générale I

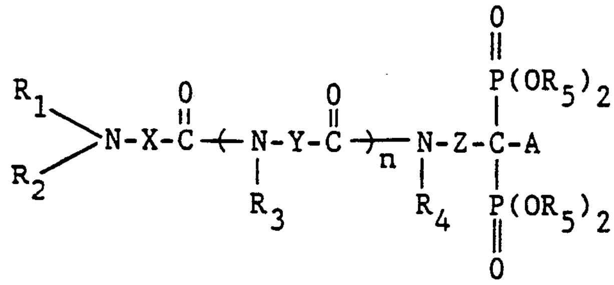

(I)

où

$R_1$, $R_2$, $R_3$, $R_4$ et $R_5$ représentent, indépendamment ou collectivement, un atome d'hydrogène ou un groupe alkyle en $C_1$—$C_4$, $R_1$ et X pouvant former, ainsi que $R_3$ et Y ou $R_4$ et Z, conjointement avec l'atome d'azote auquel ils sont liés, un cycle à cinq ou six maillons,

X et Y représentent indépendamment une chaîne alkylène linéaire ou ramifiée, comportant 1 à 6 atomes de carbone, qui peut être éventuellement substituée par des groupes aromatiques ou hétéroaromatiques,

Z représente une chaîne alkylène linéaire ou ramifiée, comportant 1 à 6 atomes de carbone, qui peut être interrompue par des hétéroatomes et éventuellement aussie substituée par des groupes aromatiques ou hétéroaromatiques,

n = 0—2 et

A = un atome d'hydrogène ou un groupe hydroxy,

ainsi que leurs sels pharmaceutiquement acceptables, caractérisé en ce qu l'on fait reagir soit

a) un diphosphonate de formule générale II

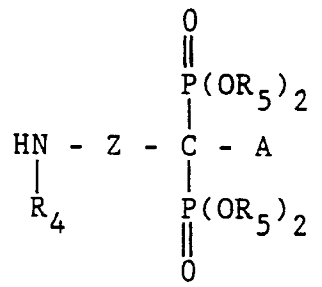

(II)

où

$R_4$ et $R_5$, Z et A ont les significations mentionnées ci-dessus, et éventuellement, les atomes d'azote présents dans le groupe Z sont protégés, avec un acide carboxylique activé, de formule générale III

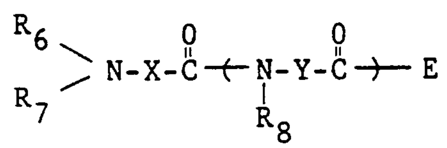

(III)

où X, Y et n ont les significations mentionnées ci-dessus, et éventuellement, les atomes d'azote présents dans ces groupes sont protégés, $R_6$, $R_7$ et $R_8$ représentent un atome d'hydrogène, un groupe alkyle inférieur ou un groupe de protection usuel dans la chimie des peptides, $R_6$ et $R_7$ pouvant également représenter conjointement le groupe phtaloyle, et $R_6$ et X ainsi que $R_8$ et Y peuvent former, conjointement avec l'atome d'azote auquel ils sont liés, un cycle à cinq ou six maillons, et E eprésente le groupe hydroxy ou un groupe d'activation usuel dans la chimie des peptides, et ensuite, on sépare les groupes de protection présents, soit

b) un composé de formule générale IV

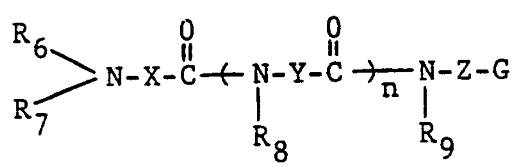

(IV)

où X, Y, Z et n ont les significations mentionnées ci-dessus, et éventuellement, les atomes d'azote présents dans ces groupes sont protégés, $R_6$, $R_7$, $R_8$ et $R_9$ représentent un atome d'hydrogène, un groupe alkyle inférieur ou un groupe de protection usuel dans la chimie des peptides, $R_6$ et $R_7$ pouvant également représenter conjointement le groupe phtaloyle et $R_6$ et X, $R_8$ et Y, ainsi que $R_9$ et Z peuvent former, conjointement avec l'atome d'azote auquel ils sont liés, un cycle à cinq ou six maillons, et G représente le groupe

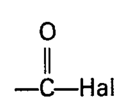

dans lequel Hal représente un atome de brome ou de chlore ou un groupe réactif comme par exemple un groupe halogéno ou sulfonate, dans le cas où G représente le groupe

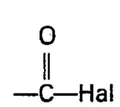

avec un phosphite de trialkyle de formule générale V

$$P(OR_{10})_3 \qquad (V),$$

où $R_{10}$ représente un groupe alkyle inférieur, pour obtenir un phosphonate d'acyle de formule générale VI

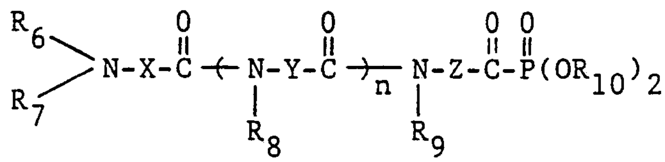

(VI),

où X, Y, Z, n, $R_6$, $R_7$, $R_8$, $R_9$ et $R_{10}$ ont les significations mentionnées ci-dessus, et ensuite, on fait reagir ce composé avec un phosphite de dialkyle de formule générale VII

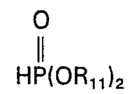

(VII),

où $R_{11}$ représente un groupe alkyle inférieur, pour obtenir un diphosphonate de formule générale VIII

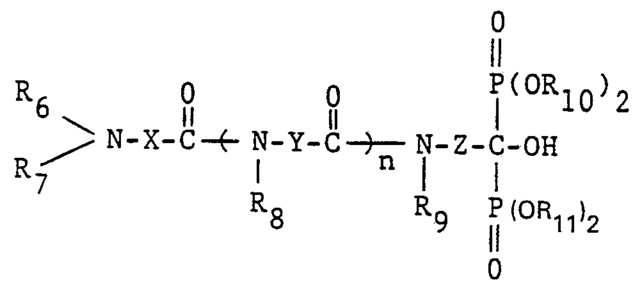

(VIII),

où X, Y, Z, n, $R_6$, $R_7$, $R_8$, $R_9$, $R_{10}$ et $R_{11}$ ont les significations mentionnées ci-dessus, ou dans le cas où G représente les groupes réactifs mentionnés, on le fait réagir avec un composé de formule générale IX

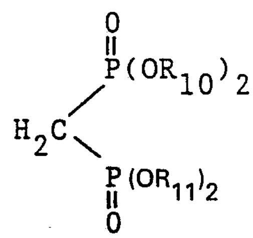

(IX)

où $R_{10}$ et $R_{11}$ ont les significations mentionnées ci-dessus, pour obtenir un diphosphonate de formule générale X

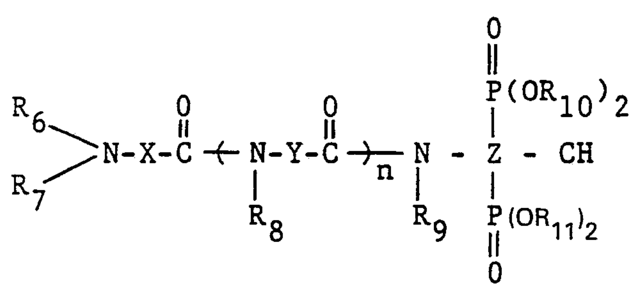

(X),

où X, Y, Z, n, $R_6$, $R_7$, $R_8$, $R_9$, $R_{10}$ et $R_{11}$ ont les significations mentionnées ci-dessus, separe le groupe de protection eventuellement présent et saponifie éventuellement le tétraester formé en diester ou acide de formule générale I ou le transforme en un sel pharmaceutiquement acceptable.

4. Procédé selon la revendication 3, caractérise en ce que l'on prépare des composés de formule I dans lesquels A représente un groupe hydroxy, n vaut 0 ou 1, $R_4$ et $R_5$ représentent un atome d'hydrogène, Z représente une chaîne alkylène lineaire comportant 2 à 5 atomes de carbone, le groupe $(R_1R_2)$—N—X—CO— représente un groupe glycyle, alanyle, prolyle ou sarcosyle, et le groupe

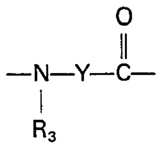

représente un groupe glycyle ou alanyle.

5. Médicament contenant un composé selon la revendication 1 et des supports et adjuvants usuels.

6. Utilisation de composés selon la revendication 1, pour la préparation de médicaments, pour le traitement des troubles du métabolisme calcique.

**Claims**

1. Diphosphonic acid derivatives of the general formula I

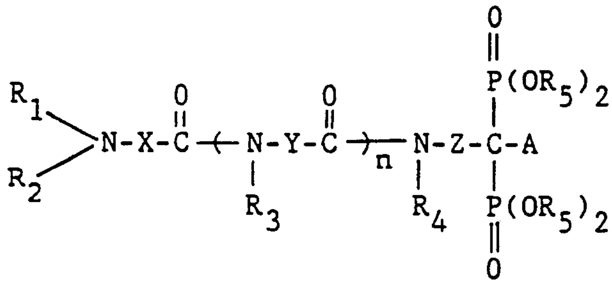

(I)

in which $R_1$, $R_2$, $R_3$, $R_4$ and $R_5$, in each case independently of one another or also together, signify hydrogen or $C_1$—$C_4$ alkyl, whereby $R_1$ and X or $R_3$ and Y or $R_4$ and Z, together with the nitrogen atom to which they are attached can form a five- or six-membered ring, X and Y, in each case independently of one another, signify a straight-chained or branched alkylene chain with 1—6 carbon atoms which can possibly be substituted by aromatic or heteroaromatics, Z signifies a straight-chained or branched alkylene chain with 1—6 carbon atoms, which can be interrupted by heteroatoms and can possibly also be substituted by aromatics or heteroaromatics, *n* = 0—2 and A = hydrogen or hydroxyl, as well as their pharmacologically acceptable salts.

2. Compounds according to claim 1, in which A signifies hydroxyl, *n* the number 0 or 1, $R_4$ and $R_5$ hydrogen, Z is a straight-chained alkylene chain with 2—5 carbon atoms, the group ($R_1R_2$)—N—X—CO— is glycyl, alanyl, prolyl or sarcosyl and the group

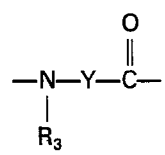

is glycyl or alanyl.

3. Process for the preparation of diphosphonic acid derivatives of the general formula I

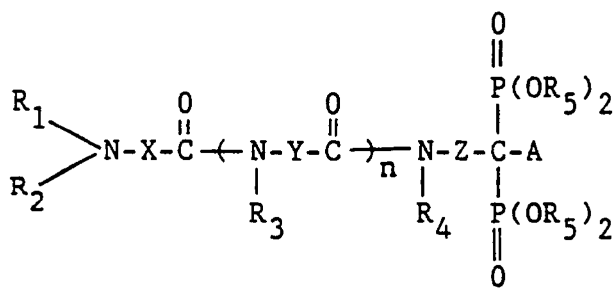

(I)

in which $R_1$, $R_2$, $R_3$, $R_4$ and $R_5$, in each case independently of one another or also together, signify hydrogen or $C_1$—$C_4$ alkyl, whereby $R_1$ and X or $R_3$ and Y or $R_4$ and Z, together with the nitrogen atom to which they are attached can form a five- or six-membered ring, X and Y, in each case independently of one another, signify a straight-chained or branched alkylene chain with 1—6 carbon atoms which can possibly be substituted by aromatic or heteroaromatics, Z signifies a straight-chained or branched alkylene chain with 1—6 carbon atoms, which can be interrupted by heteroatoms and can possibly also be substituted by aromatics or heteroaromatics, n = 0—2 and A = hydrogen or hydroxyl, as well as their pharmacologically acceptable salts, characterised in that one

a) reacts a disphosphonate of the general formula II

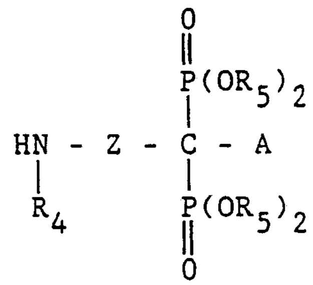

(II)

in which $R_4$, $R_5$, Z and A have the above-given meanings and nitrogen atoms possibly present in the radical Z are protected, with an activated carboxylic acid of the general formula III

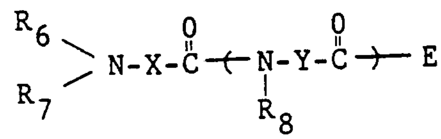

(III)

in which X, Y and *n* have the above-given meanings and nitrogen atoms possibly present in these radicals are protected, $R_6$, $R_7$ and $R_8$ represent hydrogen, lower alkyl or a protective group common in peptide chemistry, whereby $R_6$ and $R_7$ together can form the phthaloyl radical and $R_6$ and X, as well as $R_8$ and Y, together with the nitrogen atom to which they are attached, can form a five- or six-membered ring and E represents a hydroxyl or an activating group common in peptide chemistry, and subsequently splits off the protective groups present, or

b) reacts a compound of the general formula IV

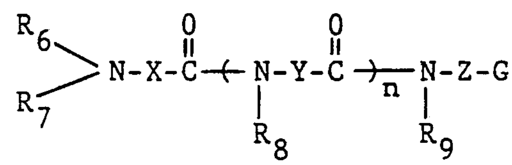

(IV)

in which X, Y, Z and *n* have the above-given meanings and nitrogen atoms possibly present in these radicals are protected, $R_6$, $R_7$, $R_8$ and $R_9$ represent hydrogen, lower alkyl or a protective group common in peptide chemistry, whereby $R_6$ and $R_7$ can together also form the phthaloyl radical and $R_6$ and X, $R_8$ and Y, as well as $R_9$ and Z, together with the nitrogen atom to which they are attached, can form a five- or six-membered ring and G signifies the group

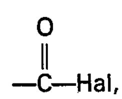

whereby Hal is to be bromine or chlorine or a reactive residue, such as e.g. halogen or sulphonate, in the case that G represents the group

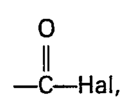

with a trialkyl phosphite of the general formula V

$$P(OR_{10})_3 \qquad (V)$$

in which $R_{10}$ signifies lower alkyl, to give an acyl phosphonate of the general formula VI

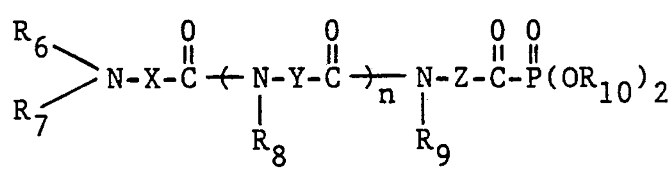

(VI),

in which X, Y, Z, *n,* $R_6$, $R_7$, $R_8$, $R_9$ and $R_{10}$ have the above-given meanings, subsequently allowed to react with a dialkyl phosphite of the general formula VII

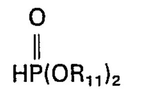

(VII),

in which $R_{11}$ signifies lower alkyl, to give a diphosphonate of the general formula VIII

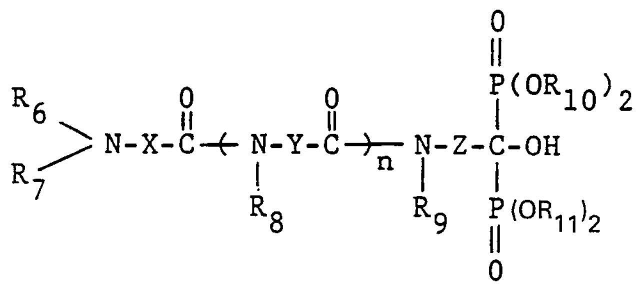

(VIII),

in which X, Y, Z, *n*, $R_6$, $R_7$, $R_8$, $R_9$, $R_{10}$ and $R_{11}$ have the above-given meanings, or in the case that G represents the above-mentioned reactive group, reacts with a compound of the general formula IX

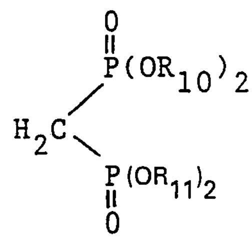

(IX)

in which $R_{10}$ and $R_{11}$ have the above-given meanings, to give a disphosphonate of the general formula X

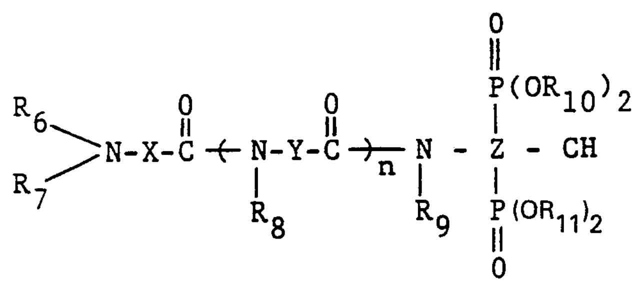

(X),

in which X, Y, Z, *n*, $R_6$, $R_7$, $R_8$, $R_9$, $R_{10}$ and $R_{11}$ have the above-given meanings, splits off the protective groups possibly present and, if desired, saponifies the resultant tetraesters to diesters or acids of the general formula I or converts into pharmacologically acceptable salts.

4. Process according to claim 3, characterised in that compounds of the formula I are prepared in which A signifies hydroxyl, *n* the number 0 or 1, $R_4$ and $R_5$ hydrogen, Z is a straight-chained alkylene chain with 2—5 carbon atoms, the group $(R_1R_2)N—X—CO—$ glycyl, alanyl, prolyl or sarcosyl radical and the group

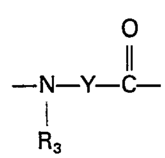

is glycyl or alanyl.

5. Medicaments containing a compound according to claim 1 and usual carrier and adjuvant materials.

6. Use of compounds according to claim 1 for the preparation of medicaments for the treatment of calcium metabolism disturbances.